(19) Europäisches Patentamt
European Patent Office
Office européen des brevets

(11) **EP 4 618 089 A1**

(12) **EUROPEAN PATENT APPLICATION**
published in accordance with Art. 153(4) EPC

(43) Date of publication:
**17.09.2025 Bulletin 2025/38**

(21) Application number: **23889114.7**

(22) Date of filing: **07.11.2023**

(51) International Patent Classification (IPC):
**G16B 5/00** (2019.01)   **G16B 20/20** (2019.01)
**G16B 40/00** (2019.01)

(52) Cooperative Patent Classification (CPC):
**G16B 5/00; G16B 20/20; G16B 40/00**

(86) International application number:
**PCT/KR2023/017786**

(87) International publication number:
**WO 2024/101856 (16.05.2024 Gazette 2024/20)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: **08.11.2022 KR 20220148030**

(71) Applicants:
• **Invites Genomics Co., Ltd.**
  **Jeju-si, Jeju-do 63243 (KR)**
• **CG Invites Co., Ltd.**
  **Seoul 07802 (KR)**

(72) Inventors:
• **CHO, Yun Sung**
  **Seongnam-si Gyeonggi-do 13539 (KR)**
• **JUN, Je Hoon**
  **Suwon-si Gyeonggi-do 16239 (KR)**
• **LEE, Hwang Yeol**
  **Yongin-si Gyeonggi-do 16953 (KR)**
• **CHUNG, Ok Sung**
  **Hwaseong-si Gyeonggi-do 18499 (KR)**

(74) Representative: **Zacco Sweden AB**
  **P.O. Box 5581**
  **Löjtnantsgatan 21**
  **114 85 Stockholm (SE)**

(54) **METHOD FOR PREDICTING IMMUNOGENICITY OF NEOANTIGEN EPITOPE, AND DEVICE USING SAME**

(57)    A method for predicting the immunogenicity of a mutant epitope, comprising: calculating the degree to which each of factors involved in a biological process for a mutant epitope and properties of the epitope affects the immunogenicity of the mutant epitope; calculating the degree to which each of factors involved in a biological process for a wild-type epitope corresponding to the mutant epitope and properties of the epitope affects the immunogenicity of the wild-type epitope; calculate a differential index based on a value calculated for the mutant epitope and a value calculated for the wild-type epitope; and inputting the value calculated for the mutant epitope and the calculated differential index into a pre-trained artificial intelligence model to predict the immunogenicity of the mutant epitope.

**FIG. 4**

Calculating degree to which each of factors involved in biological process for mutant epitope and properties of epitope affects immunogenicity of mutant epitope — S410

Calculating degree to which each of factors involved in biological process for wild-type epitope corresponding to mutant epitope and properties of epitope affects immunogenicity of wild-type epitope — S420

Calculating differential index based on value calculated for mutant epitope and value calculated for wild-type epitope — S430

Inputting value calculated for mutant epitope and calculated differential index into pre-trained artificial intelligence model to predict immunogenicity of mutant epitope — S440

EP 4 618 089 A1

## Description

## Technical Field

The present disclosure relates to a method for predicting the immunogenicity of a neoantigen epitope, and a device using the same, and more particularly, to a method for predicting the immunogenicity of a neoantigen epitope by comparing a mutated neoantigen epitope with a wild-type epitope, and a device using the same.

## Background Art

Neoantigens are mutated antigens that are expressed in tumor cells/tissues and is not expressed in normal cells. When a normal sequence is altered by mutation, immune cells recognize an altered sequence as a new antigen/epitope and elicit an immune response. These neoantigens are utilized as therapeutic vaccines and are used in immunotherapy and predicting reactivity. Various factors involved in many biological processes such as MHC binding affinity, proteasomal cleavage, TAP transporter efficiency, pMHC stability, and T-cell receptor interaction, and properties of neoantigen epitopes such as hydrophobicity, similarity to known epitopes, and dissimilarity to self-proteome may affect the prediction of neoantigen epitopes that exhibit CD8+ T-cell immunogenicity in silico. Therefore, using various factors involved in biological processes caused by mutations and/or the properties of neoantigen epitopes, the immunogenicity of neoantigen epitopes was predicted.

## Summary of Invention

## Technical Problem

Up to now, methods have been developed to predict epitopes that induce an immune response by individually utilizing various factors involved in many biological processes and/or epitope properties, or by combining a small number of factors and/or properties. Differential agretopicity index (hereinafter referred to as DAI) is also one of the factors used to predict the immunogenicity of an epitope.
DAI is defined as a ratio of the MHC affinity of a mutant epitope (which may also be referred to as a "peptide") to the MHC affinity of a non-mutated or wild-type epitope. It may be more effective to predict immunogenicity using DAI rather than using only the MHC affinity of a neoepitope or mutant epitope. The reason is that not all mutant epitopes exhibit immunogenicity simply because they are favored by certain factors such as MHC binding affinity. Even if the wild-type epitope itself has a tendency to bind well to MHC, it can act to prevent an immune response against a self-epitope through the immune system's negative selection/central tolerance process etc., that is, to prevent normal cells from being attacked by immune cells. Therefore, measuring the difference in how the properties are changed by mutation compared to a normal, wild-type epitope may be very important in predicting the immunogenicity of an epitope.
The difference between the mutant epitope and the wild-type epitope may not only affect the binding affinity, but may also affect various biological processes involved in eliciting an immune response by the epitope. For example, while the MHC binding affinity of the wild-type epitope and the mutant epitope is similar, the efficiency/response of proteasomal cleavage, TAP transporter efficiency, pMHC stability, T-cell receptor interaction, etc. may differ due to mutation.
Therefore, the present disclosure proposes a method for predicting the immunogenicity of a mutant epitope using a differential index (i.e., a ratio or difference between the measured values of a mutant epitope and a wild-type epitope) for many biological processes and epitope/neoepitope properties, and a device using the same.

## Solution to Problem

According to some aspects of the disclosure, a method for predicting the immunogenicity of a mutant epitope, comprises: calculating the degree to which each of factors involved in a biological process for a mutant epitope and properties of the epitope affects the immunogenicity of the mutant epitope, calculating the degree to which each of factors involved in a biological process for a wild-type epitope corresponding to the mutant epitope and properties of the epitope affects the immunogenicity of the wild-type epitope, calculate a differential index based on a value calculated for the mutant epitope and a value calculated for the wild-type epitope; and inputting the value calculated for the mutant epitope and the calculated differential index into a pre-trained artificial intelligence model to predict the immunogenicity of the mutant epitope.
According to some aspects, further comprising performing at least one of standardization and normalization on the value calculated for the mutant epitope and the calculated differential index before inputting the calculated value into the trained artificial intelligence model.
According to some aspects, the differential index includes at least one of RATIO between the value calculated for the mutant epitope and the value calculated for the wild-type epitope and DIFFERENCE between the value calculated for the mutant epitope and the value calculated for the wild-type epitope.

According to some aspects, the differential index is a differential index for at least one of phosphorylation, hydrophobicity, similarity to known epitopes, dissimilarity to self-proteome, epitope stability, antigen processing, MHC I binding affinity, pMHC stability, immunogenicity, inflammatory response, B-cell linear epitope, and B-cell conformational epitope.

According to some aspects, the calculating of the degree to which each of the factors involved in the biological processes for the mutant epitope and the wild-type epitope and the properties of the epitope affects the immunogenicity comprises calculating the degree using an algorithm, a program, or a tool.

According to some aspects, the biological process comprises an antigen processing stage, an antigen presentation stage, an immunogenicity stage, and a tumor microenvironment, and wherein the factors involved in the tumor microenvironment are at least one of inflammatory response, B-cell linear epitope, and B-cell conformational epitope.

According to some aspects of the disclosure, a computer-readable recording medium recording a computer program for executing the method of any one of claims 1 to 6.

According to some aspects of the disclosure, a device for predicting the immunogenicity of a mutant epitope, comprises: an input/output device receiving a mutant epitope or outputting a prediction result of the immunogenicity of the mutant epitope; a storage device storing an artificial intelligence model trained to predict the immunogenicity of a mutant epitope using a differential index calculated based on factors involved in biological processes for the mutant epitope and a wild-type epitope and properties of the epitope, and a value calculated based on the factors involved in the biological process for the mutant epitope and the properties of the epitope; and a computing device calculating the degree to which each of the factors involved in the biological process for the mutant epitope and the properties of the epitope affects the immunogenicity of the mutant epitope, calculating the degree to which each of the factors involved in the biological process for the wild-type epitope corresponding to the mutant epitope and the properties of the epitope affects the immunogenicity of the wild-type epitope, calculating the differential index based on a value calculated for the mutant epitope and a value calculated for the wild-type epitope, and inputting the value calculated for the mutant epitope and the calculated differential index into a pre-trained artificial intelligence model to predict the immunogenicity of the mutant epitope.

**Advantageous Effects**

According to the present disclosure, since it is possible to consider various aspects of many biological processes and epitope/neoepitope properties caused by mutations, it is possible to predict the immunogenicity of a mutant epitope with higher accuracy than when immunogenicity is predicted using only the mutant epitope. The immunogenic epitopes predicted with high accuracy can be expected to have higher efficacy and lower side effects when developing a neoantigen-based vaccine.

**BRIEF DESCRIPTION OF THE DRAWINGS**

FIG. 1 illustrates an example of a biological process of a tumor cell.

FIG. 2 illustrates factors of many biological processes that may affect the immunogenicity of an epitope and the epitope properties.

FIG. 3 illustrates an example of a process for learning the immunogenicity of a mutant epitope using an artificial intelligence model.

FIG. 4 illustrates an example of a process for predicting the immunogenicity of a mutant epitope using an artificial intelligence model.

FIG. 5 illustrates an example of a configuration diagram of a device for predicting the immunogenicity of a mutant epitope using an artificial intelligence model.

**DETAILED DESCRIPTION**

The following embodiments are only for explaining the present disclosure more specifically, and it will be obvious to those skilled in the art that the scope of the present disclosure is not limited by these examples according to the gist of the present disclosure. It should be understood that all modifications, equivalents, or substitutes included in the spirit and scope of the technology described below are included.

In the terms used herein, expressions of the singular should be understood to include the plural unless the context clearly indicates otherwise. The term "includes" and the like should be understood to mean the presence of the described features, numbers, steps, operations, components, parts, or combinations thereof, and not to exclude the possibility of the presence or addition of one or more other features, numbers, steps, operations, components, parts, or combinations thereof. The term and/or includes a combination of a plurality of related recited items or any one of a plurality of related recited items.

In performing a method or method of operation, each process/step constituting the method may occur differently from the stated order unless the context clearly indicates a particular order. That is, each processes/step may occur in the same order as stated, may be performed substantially simultaneously, or may be performed in reverse order.

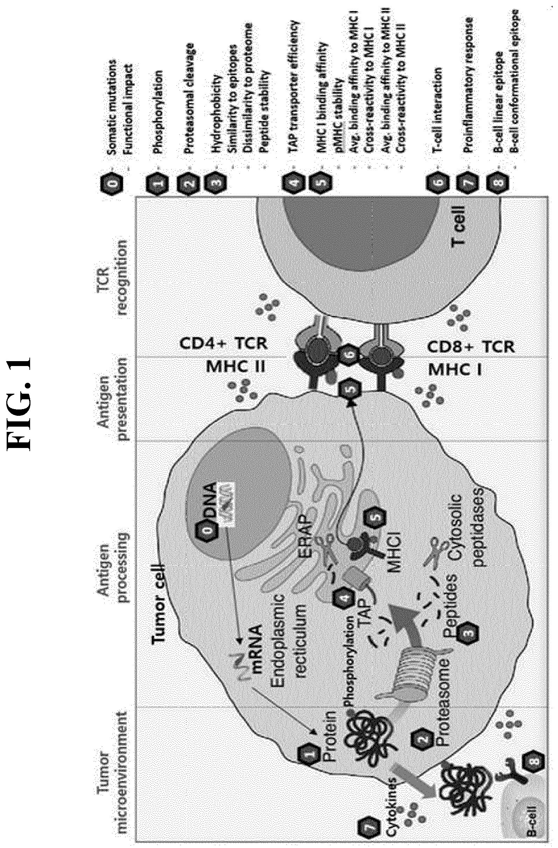

FIG. 1 illustrates an example of a biological process of a tumor cell.

Referring to FIG. 1, the biological process of a tumor cell may comprise an antigen processing stage, an antigen presentation stage, and an immunogenicity stage. A tumor microenvironment (TME) surrounding the tumor cell may also affect the biological process, and thus may be considered as a part of the biological process of the tumor cell. The biological process of the tumor cell is the same as or similar to the biological process of normal cells, but various factors may affect the generation and/or properties of epitopes at each stage.

The antigen processing stage is a stage in which DNA in the nucleus of a tumor cell is transcribed into mRNA and released into the cytoplasm, and the transcribed mRNA is transformed into a peptide and binds to MHC class I. The DNA of the tumor cell may be transformed due to mutations, etc. The mRNA may be translated and synthesized into a protein, and then fragmented into a peptide by the proteasome. The fragmented peptide is transported into an endoplasmic reticulum by a TAP transporter and binds to MHC class I.

The antigen presentation stage is a stage in which the peptide generated in the antigen processing stage binds to MHC class I and is presented on the cell surface as a peptide-MHC complex.

The immunogenicity stage is a stage in which the peptide-MHC complex presented on the cell surface is recognized by T cells.

The tumor microenvironment (TME) is an environment surrounding a cell, and the cell may be surrounded by blood vessels, immune cells, fibroblasts, bone marrow-derived inflammatory cells, lymphocytes, signaling molecules, and extracellular matrix. Tumor cells may affect the tumor microenvironment by inducing extracellular signal release, tumor cell angiogenesis promotion, and peripheral immune tolerance, and the tumor microenvironment may also affect the growth and/or metastasis of tumor cells. For example, if the disease that induces an immune response is cancer, the clonality of a sample may affect the immunogenicity of an epitope, and the distribution/proportion of immune cells, tumor infiltrating lymphocytes, RNA expression, etc. may also affect the immunogenicity of the generated epitope.

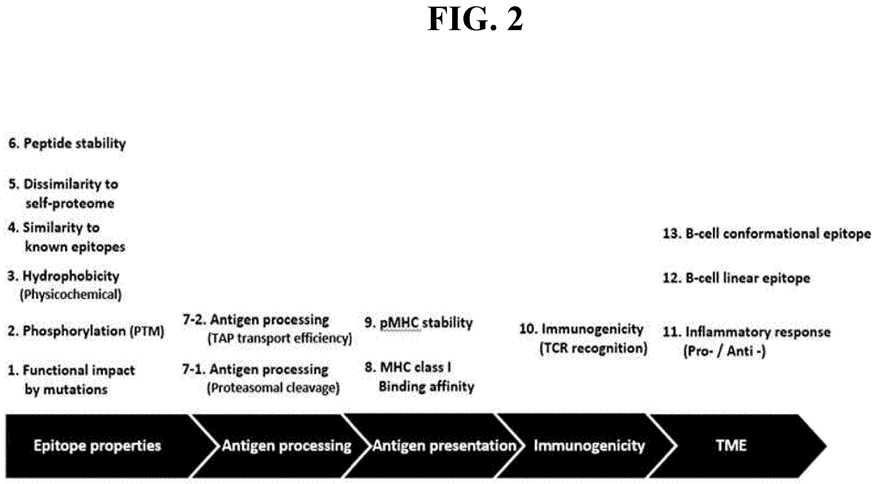

FIG. 2 illustrates factors of many biological processes that may affect the immunogenicity of an epitope and the epitope properties.

In addition to the biological processes described in FIG. 1, the immunogenicity of an epitope may also be affected by the properties of the epitope. For example, negative factors affecting the human body, such as toxicity and allergenicity of the epitope, may affect the immunogenicity of an epitope. The factors affecting the immunogenicity of an epitope may affect one at a time, but may also affect multiple factors simultaneously and/or sequentially.

Hereinafter, the epitope properties and the various factors that may affect the immunogenicity of an epitope in the biological process will be described in detail.

Among epitope properties, factors that may affect immunogenicity include functional impact by mutations, phosphorylation (one of PTM), hydrophobicity (or physicochemical hydrophobicity), similarity to known epitopes, dissimilarity to self (human reference)-proteome, peptide stability, etc.

Hereinafter, specific programs/algorithms/tools are described as examples to confirm the degree to which each factor affects the immunogenicity of an epitope, but are not limited thereto.

The functional impact by mutations is a factor that indicates the impact on the function of a protein due to somatic mutations that cause tumors. Epitopes containing mutations with large functional impact are naturally eliminated by existing immune cells, but epitopes containing mutations with relatively small functional impact may exhibit immunogenicity at a higher frequency. Programs such as PROVEAN and/or PolyPhen-2 may be used to confirm the degree to which the functional impact by mutations affects the immunogenicity of an epitope.

Phosphorylation is a factor that indicates the degree to which the MHC class I ligand of an epitope for which immunogenicity is to be predicted is phosphorylated. A program such as NetMHCphosPan may be used to confirm the degree to which phosphorylation affects the immunogenicity of an epitope.

Hydrophobicity is a factor that indicates whether the epitope for which immunogenicity is to be predicted is hydrophobic. A program such as ProtFP may be used to confirm the degree to which hydrophobicity affects the immunogenicity of an epitope.

Similarity to known epitopes is a factor that indicates whether the epitopes for which immunogenicity is to be predicted are similar to epitopes known to be immunogenic (or non-immunogenic). A program such as blastP may be used to confirm the degree to which the similarity to known epitopes affects the immunogenicity of an epitope.

The dissimilarity to self (human reference)-proteome is a factor that indicates whether the epitope for which immunogenicity is to be predicted is dissimilar to a self-proteome. Here, the self-proteome may be a human-referenced self-proteome. For example, the pairwise2 module of Python Bio package may be used to confirm the degree to which dissimilarity to self (human reference)-proteome affects the immunogenicity of an epitope.

Epitope stability (or peptide stability) is a factor that indicates the stability of the epitope itself. Previously, the stability of peptide-MHC binding was used, but the stability of the epitope itself was not used. If the stability of the epitope is low, the opportunity to bind/interact with the MHC molecule is reduced, so the possibility of having immunogenicity may be low. A program such as ProtParam may be used to confirm the degree to which epitope stability affects the immunogenicity of an epitope.

In the antigen processing stage of the biological process, proteasomal cleavage and/or TAP transporter efficiency may be used to predict the immunogenicity of an epitope. A program such as NetCTLpan may be used to confirm the degree to which proteasomal cleavage and TAP transporter efficiency affect the immunogenicity of an epitope.

In the antigen presentation stage of the biological process, at least some of the MHC I binding affinity and/or pMHC stability may be used to predict an epitope that elicits an immune response.

MHC class I binding affinity is a factor that indicates the degree to which an epitope for which immunogenicity is to be predicted binds to MHC class I. Programs such as NetMHCpan and/or MHCflurry may be used to confirm the degree to which MHC class I binding affinity affects the immunogenicity of an epitope.

pMHC stability is a factor that indicates the peptide-MHC stability of an epitope. A program such as NetMHCstabpan may be used to confirm the degree to which pMHC stability affects the immunogenicity of an epitope.

In the immunogenicity stage of the biological process, immunogenicity may be used to predict an epitope that elicits an immune response. At least one of programs such as IEDB, PRIME, DeepHLApan, and DeepImmuno may be used to confirm the degree to which immunogenicity affects the immunogenicity of an epitope.

In the tumor microenvironment, at least one of inflammatory response, B-cell linear epitope, and B-cell conformational epitope may be considered as a factor predicting an epitope that elicits an immune response.

Immune cells may regulate an immune response by secreting inflammatory substances such as cytokines. The ability of an epitope to induce these cytokines is calculated, and if the epitope induces cytokines excessively/insufficiently, the immune response may be suppressed. At least one of the programs such as PIP-EL, AIPpred, and IFNepitope may be used to confirm the degree to which the inflammatory response affects the immunogenicity of an epitope.

Somatic mutations that cause tumors may be recognized by B-cells. B-cells may recognize both linear epitopes in the form of peptides, and conformational epitopes in the form of protein structures. B-cell linear epitopes are factors that indicate linear epitope potential, and B-cell confirmational epitopes are factors that indicate conformational epitope potential. For example, the Bepipred program may be used to conform the degree to which B-cell linear epitopes affect the immunogenicity of an epitope. For example, Discotope may be used to confirm the degree to which B-cell conformational epitopes affect the immunogenicity of an epitope.

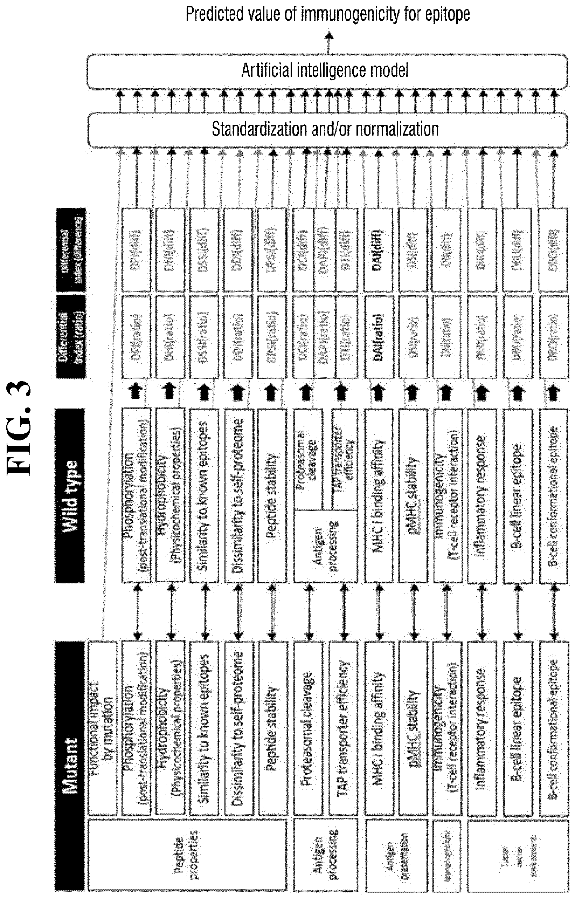

FIG. 3 illustrates an example of a process for learning the immunogenicity of a mutant epitope using an artificial intelligence model.

In order to learn and evaluate the CD8+ T-cell immunogenicity of neoantigens, immunogenic mutant epitopes and non-immunogenic mutant epitopes may be used. The immunogenic mutant epitopes and non-immunogenic mutant epitopes may be previously published data and/or data owned and/or produced internally. Previously published data may typically be obtained using dbPepNeo, NEPdb, etc., and since it is necessary to calculate a differential index between the mutant epitope and the wild-type epitope, data may be collected only for tumor neoantigen epitopes. That is, pathogen-derived epitopes, from which information on the mutant epitope and the wild-type epitope may not be directly or indirectly extracted, may be excluded because the differential index may not be calculated.

The factors involved in numerous biological processes, which affect the immunogenicity of the mutant epitope, and the properties of the mutant epitope include the properties of the mutant epitope, antigen processing, antigen presentation, immunity (or T-cell recognition), tumor microenvironment (TME), etc., and specifically,, functional impact by mutations, PTMs (phosphorylation, ubiquitination, glycosylation, etc.), sequence similarity to known epitopes, dissimilarity to self-proteome, epitope (peptide) stability, proteasomal cleavage, TAP transporter efficiency, MHC class I/II binding affinity, pMHC stability, immunity (T-cell receptor recognition), inflammatory response, B-cell linear epitopes, B-cell conformational epitopes, etc. If the disease to be treated is cancer, the clonality of the sample may be further included, and TME factors other than inflammation (distribution/ratio of immune cells, tumor-infiltrating lymphocytes, RNA expression, etc.) may also be included. In addition, negative factors affecting the human body, such as toxicity and/or allergenicity of the epitope, may also be further included. According to an embodiment, many biological processes and epitope properties may be partially selected and utilized.

Among the detailed factors involved in the immunogenicity of the mutant epitope, the factors that may calculate the differential index may be at least one of phosphorylation, hydrophobicity, similarity to known epitopes, dissimilarity to self-proteome, epitope stability, antigen processing, MHC I binding affinity, pMHC stability, immunogenicity, inflammatory response, B-cell linear epitope, and B-cell conformational epitope. The functional impact by mutations is excluded from the factors that may be used to calculate the differential index, because the value measuring the functional impact by mutations itself measures the effect of the mutant epitope on the protein compared to the wild-type epitope. According to an embodiment, antigen processing may be one factor, but it may also be divided into two factors: proteasomal cleavage and TAP transporter efficiency.

The learning may be performed simultaneously in an integrated manner for all factors or performed separately for each factor. For example, if data matching all factors is insufficient, the learning may be performed only for factors on which data exist. The data to be input for the learning may be values of each factor measured using a program/algorithm/tool. The program/algorithm/tool may be at least one of those described in FIG. 2 or may be developed separately. If one factor is measured using multiple programs/algorithms/tools, each of the measured factors may be input. In another example, multiple factors may be measured using one program/algorithm/tool and input.

The immunogenic/non-immunogenic epitopes to be used for learning are composed of a pair of mutant epitopes and wild-type epitopes, and the immunogenicity of the mutant epitopes may be predicted by using the values derived for the mutant epitopes and wild-type epitopes for each factor using the collected or developed algorithm/tool.

In the present disclosure, the differential index of the mutant epitopes and wild-type epitopes for each factor may be calculated as RATIO and DIFFERENCE and used as input for learning, and one or both may be used. The following [Equation 1] and [Equation 2] represent RATIO and DIFFERENCE as Equations:

[Equation 1]

$$RATIO = \frac{\text{Value measured using program/algorithm/tool for mutant epitope}}{\text{Value measured using program/algorithm/tool for wile-type epitope}}$$

DIFFERENCE = Value measured using program/algorithm/tool for mutant epitope - Value measured using program/algorithm/tool for wild-type epitope     [Equation 2]

In other embodiments, in addition to [Equation 1] and [Equation 2], a value obtained by applying LOG, which is a derivative of the basic equation, may be used.

Differential indices for various factors involved in many biological processes, which affect the immunogenicity of the mutant epitope, and the properties of the mutant epitope may be named as follows, but are not limited thereto.

- Phosphorylation (one of PTM): Differential phosphorylation index (DPI)
- Hydrophobicity: Differential hydrophobicity index (DHI)
- Similarity to known epitopes: Differential sequence similarity index (DSSI)
- Dissimilarity to self-proteome: Differential dissimilarity index (DDI)
- Peptide (epitope) stability: Differential peptide stability index (DPSI)
- Proteasomal cleavage: Differential cleavage index (DCI)
- TAP transport efficiency: Differential TAP index (DTI)
- Antigen processing: Differential antigen processing index (DAPI)
- MHC I binding affinity: Differential agretopicity index (DAI)
- pMHC stability: Differential stability index (DSI)
- Immunogenicity (T-cell receptor interaction): Differential immunogenicity index (DII)
- Inflammatory response (inflammatory response): Differential inflammatory response index (DIRI)
- B-cell linear epitope: Differential B-cell linear epitope index (DBLI)
- B-cell conformational epitope: Differential B-cell conformational epitope index (DBCI)

Statistically significant factors among values obtained by measuring factors involved in various biological processes and properties of the epitope using a program/algorithm/tool for a mutant epitope and values calculated using [Equation 1] and [Equation 2] are selected, encoded, and machine-learned or learned in a statistical manner. Before the encoding is performed, standardization and/or normalization may be performed on each of the factors and the values calculated using [Equation 1] and [Equation 2]. The values derived using the program/algorithm/tool may have different value ranges or resolutions depending on each of factors or the applied program/algorithm/tool, and thus may be standardized and/or normalized and used.

The RATIO and/or DIFFERENCE for each factor may be selected and utilized in whole or in part through statistical evaluation. For example, the R ATIO and DIFFERENCE for all factors may be used simultaneously for learning, and/or statistical significance may be examined/evaluated among them, and only partially significant values may be used for learning. In an embodiment, the addition/subtraction of the program/algorithm/tool and/or individual factors may vary depending on the target disease. For example, different factors and/or combinations of programs/algorithms/tools may be formed depending on the type of cancer.

The artificial intelligence models that may be used for learning include neural networks, deep learning, logistic regression, AdaBoost, LogitBoost, XgBoost, Support vector machines, and random forests, and are not limited to specific machine learning algorithms or statistical methods. In addition, ensemble machine learning methods (e.g., bagging, stacking, voting, boosting, etc.) that integrate multiple machine learning algorithms or statistical methods may also be utilized. By integrating learning using these machine learning methods, the weights of the factors that predict the final goal, the immunogenic mutant epitopes, are determined, and learning is possible by utilizing the factors involved in all biological processes, the properties of the epitopes, and the differences between the mutant epitopes and the non-mutated epitopes.

The artificial intelligence models generated through the above learning may be evaluated for performance through existing known cross validation, leave-one-out validation, or validation using independent data, and the performance may be evaluated experimentally *in vitro, in vivo,* and clinically for the predicted mutant epitopes.

The result of the artificial intelligence model is a predicted value of immunogenicity for the mutant epitope. The predicted value of immunogenicity for the mutant epitope is a probability that the epitope is immunogenic or non-immunogenic, and may be a value between 0 and 1. For example, if the predicted value of immunogenicity for the mutant epitope is close to 1, it may be judged as immunogenic, and if the predicted value of immunogenicity for the mutant epitope is close to 0, it may be judged as non-immunogenic.

According to another embodiment, the result of the artificial intelligence model may be a result of determining whether the mutant epitope is immunogenic. The artificial intelligence model may compare the predicted value of immunogenicity for the mutant epitope with a reference value and determine it as immunogenic or non-immunogenic. The reference value may vary depending on any one or a combination of the tumor type, purpose, and situation.

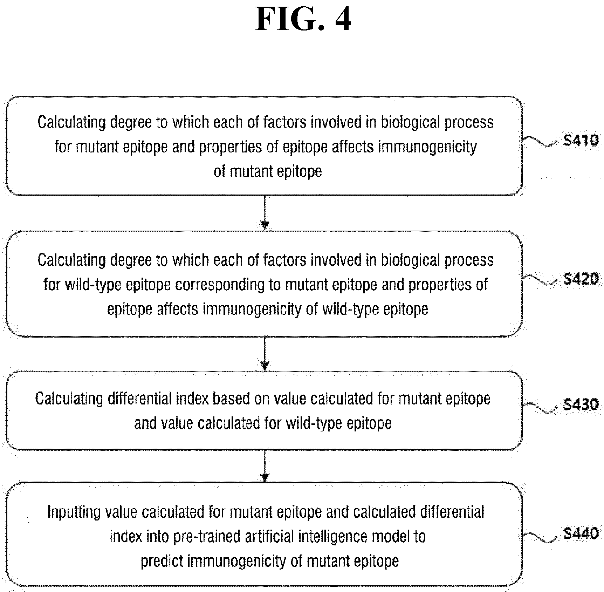

FIG. 4 illustrates an example of a process for predicting the immunogenicity of a mutant epitope using an artificial intelligence model.

Referring to FIG. 4, a method for predicting the immunogenicity of a mutant epitope according to an embodiment of the present disclosure may include a step/process (S410) of calculating the degree to which each of factors involved in a biological process for a mutant epitope and properties of the epitope affects the immunogenicity of the mutant epitope.

A method for predicting the immunogenicity of a mutant epitope may include a step/process (S420) of calculating the degree to which each of factors involved in a biological process for a non-mutated epitope corresponding to the mutant epitope and properties of the epitope affects the immunogenicity of the wild-type epitope.

A method of predicting the immunogenicity of a mutant epitope may include a step/process (S430) of calculating a differential index based on a value calculated for the mutant epitope and a value calculated for the wild-type epitope. The differential index may include at least one of RATIO between a value calculated for the mutant epitope and a value calculated for the wild-type epitope, and DIFFERENCE between the value calculated for the mutant epitope and the value calculated for the wild-type epitope, and may be a differential index for at least one of phosphorylation, hydrophobicity, similarity to known epitopes, dissimilarity to self-proteome, epitope stability, antigen processing, MHC I binding affinity, pMHC stability, immunogenicity, inflammatory response, B-cell linear epitope, and B-cell conformational epitope.

A method for predicting the immunogenicity of a mutant epitope may include a step/process (S440) of inputting the value calculated for the mutant epitope and the calculated differential index into a pre-trained artificial intelligence model to predict the immunogenicity of the mutant epitope. The method for predicting the immunogenicity of a mutant epitope may further include: performing at least one of standardization and normalization on the value calculated for the mutant epitope and the calculated differential index before inputting the calculated value into the trained artificial intelligence model.

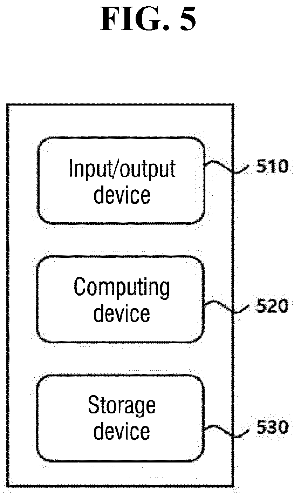

FIG. 5 illustrates an example of a configuration diagram of a device for predicting the immunogenicity of a mutant epitope using an artificial intelligence model.

Referring to FIG. 5, a device for predicting the immunogenicity of a mutant epitope using an artificial intelligence model may include an input/output device 510, a storage device 520, and a computing device 530.

The input/output device 510 (or an input and output device) may be configured to receive a mutant epitope or output a prediction result of the immunogenicity of the mutant epitope. The input/output device 510 may be configured to receive a mutant epitope from a user or another device. Here, the input/output device 510 has been illustrated as one device, but may be configured as separate devices. For example, the input device may be a mouse and/or a keyboard, and the output device may be a display such as a monitor or a speaker.

The storage device 520 may store an artificial intelligence model trained to predict the immunogenicity of a mutant epitope using a differential index calculated based on factors involved in the biological processes for the mutant epitope and a wild-type epitope and the properties of the epitope, and a value calculated based on the factors involved in the biological process for the mutant epitope and the properties of the epitope. The storage device 520 may store a programs/algorithms/tool required for data processing in addition to the artificial intelligence learning model.

The computing device 530 may calculate the degree to which each of the factors involved in the biological process for the mutant epitope and the properties of the epitope affects the immunogenicity of the mutant epitope, may calculate the degree to which each of the factors involved in the biological process for the wild-type epitope corresponding to the mutant epitope and the properties of the epitope affects the immunogenicity of the wild-type epitope, may calculate the differential index based on a value calculated for the mutant epitope and a value calculated for the wild-type epitope, and may input the value calculated for the mutant epitope and the calculated differential index into a pre-trained artificial intelligence model to predict the immunogenicity of the mutant epitope.

The computational device 530 may further perform at least one of standardization and normalization on the value calculated for the mutant epitope and the calculated differential index before inputting the calculated value into the trained artificial intelligence model.

Here, the device for predicting the immunogenicity of a mutant epitope using an artificial intelligence model is described as comprising an input/output device 510, a storage device 520, and a computing device 530. However, a plurality of configurations may be integrated into one configuration, or a single configuration may be divided inti a plurality of configurations. In addition, a device for predicting the immunogenicity of a mutant epitope using an artificial intelligence model may further include a communication device, etc.

The differential index may include at least one of RATIO between a value calculated for the mutant epitope and a value calculated for the wild-type epitope, and DIFFERENCE between a value calculated for the mutant epitope and a value calculated for the wild-type epitope, and may be a differential index for at least one of phosphorylation, hydrophobicity, similarity to known epitopes, dissimilarity to self-proteome, epitope stability, antigen processing, MHC I binding affinity, pMHC stability, immunogenicity, inflammatory response, B-cell linear epitope, and B-cell conformational epitope.

The following shows an example of predicting the immunogenicity of mutant epitopes according to the method described above.

In order to learn and evaluate the immunogenicity of epitopes, data on immunogenic epitopes and non-immunogenic epitopes for neoepitopes that occur in tumors were collected from public databases and/or papers, etc.

[Table 1] below shows the number of training data sets (data sets for training) and evaluation data sets (data sets for evaluation).

[Table 1]

| Category | CD8+ T-cell immunogenic epitopes (positive) | CD8+ T-cell non-immunogenic epitopes (negative) |
|---|---|---|
| Training data sets | 315 | 4067 |
| Evaluation data sets | 79 | 3125 |

In this Example, as shown in [Table 1], a total of 315 immunogenic epitopes (positive) and 4067 non-immunogenic epitopes (negative) (i.e., epitopes and MHC I alleles binding to the epitopes) collected from dbPepNeo, NEPdb, PRIME data, and INeo-Epp data were used as training data sets, and 79 immunogenic epitopes (positive) and 3125 non-immunogenic epitopes (negative) collected from McPAS-TCR, VDJdb, IEDB t-cell db, and TESLA consortium data were used as an evaluation data sets (independent sets). To predict the immunogenicity of epitopes, the factors involved in each biological process and the epitope properties were calculated for the mutant epitopes and the wild-type epitopes using the programs and methods presented in [Table 2].

[Table 2]

| Biological process/ Epitope properties | Program/Algorithm/ Tool | References |
|---|---|---|
| Functional impact by the mutation | PROVEAN | PMID: 23056405 |
| Phosphorylation (phosphorylated MHC class I ligands) | NetMHCphosPan | 10.1016/j.immuno.2021.100005 |
| Hydrophobicity | ProtFP | PMID: 24059694 |
| Similarity to known epitopes | blastP | - |
| Dissimilarity to self (human reference)-proteome | "pairwise2" module in Python Bio package | - |
| Peptide stability | ProtParam | PMID: 2075190 |
| Proteasomal cleavage | NetCTLpan | PMID: 20379710 |
| TAP transporter efficiency | NetCTLpan | PMID: 20379710 |

(continued)

| Biological process/ Epitope properties | Program/Algorithm/ Tool | References |
|---|---|---|
| Antigen processing (Proteasomal cleavage + TAP transporter efficiency) | MHCflurry (antigen processing score) | PMID: 32711842 |
| MHC I binding affinity | NetMHCpan | PMID: 32406916 |
| | MHCflurry (antigen presentation score) | PMID: 32711842 |
| pMHC stability | NetMHCstabpan | PMID: 27402703 |
| Immunogenicity (T-cell interaction) | IEDB tools | PMID: 24204222 |
| | PRIME | PMID: 33665637 |
| Inflammatory response | PIP-EL | PMID: 30108593 |
| B-cell linear epitope | Bepipred | PMID: 16635264 |
| B-cell conformational epitope | Discotope | PMID: 17001032 |

Afterwards, the DIFFERENCE and RATIO described above are calculated based on the values calculated by the program and method presented in [Table 2] for the mutant epitopes and the wild-type epitopes, respectively. The discriminatory ability to distinguish between immunogenic epitopes and non-immunogenic epitopes was confirmed based on AUC, prAUC, and P-value (Wilcoxon rank-sum test, $P < 0.05$) using at least one of the values calculated by the program and method presented in [Table 2] for the mutant epitopes, DIFFERENCE, and RATIO. Among these, only statistically significant factors were input into the artificial intelligence learning model.

[Table 3]

| Methods/Programs | Epitope | | | DIFFERENCE | | | RATIO | | |
|---|---|---|---|---|---|---|---|---|---|
| | AUC | prAUC | P-value | AUC | prAUC | P-value | AUC | prAUC | P-value |
| Similarity to known epitopes | 0.5610 52 | 0.142 87 | 2.91E -04 | 0.518 993 | 0.082 119 | 0.261 265 | 0.523 862 | 0.079 501 | 0.158 083 |
| Dissimilarity to self-proteome | 0.4590 67 | 0.066 936 | 0.015 349 | 0.458 521 | 0.065 998 | 0.014 032 | 0.541 067 | 0.081 895 | 0.015 003 |
| Hydrophobicity (ProtFP) | 0.5500 65 | 0.078 362 | 0.003 028 | 0.538 348 | 0.086 689 | 0.022 478 | 0.531 443 | 0.078 767 | 0.062 56 |
| NetCTLpan (Cleavage) | 0.5352 54 | 0.083 073 | 0.036 788 | 0.489 895 | 0.072 992 | 0.552 856 | 0.492 999 | 0.071 916 | 0.682 194 |
| NetCTLpan (TAP efficiency) | 0.5260 48 | 0.079 180 | 0.122 752 | 0.501 619 | 0.075 759 | 0.806 286 | 0.489 829 | 0.071 463 | 0.650 069 |
| NetCTLpan (Comb) | 0.5617 6 | 0.126 133 | 2.55E -04 | 0.517 495 | 0.079 112 | 0.300 154 | 0.513 233 | 0.072 897 | 0.433 22 |
| NetMHCpan (%Rank_EL) | 0.6522 55 | 0.167 535 | 1.90E -19 | 0.506 969 | 0.069 157 | 0.679 652 | 0.544 356 | 0.105 322 | 0.008 624 |
| NetMHCpan (%Rank_BA) | 0.6577 36 | 0.148 926 | 9.41E -21 | 0.499 05 | 0.068 368 | 0.955 226 | 0.562 486 | 0.099 488 | 2.15E -04 |
| MHCflurry (processing score) | 0.6502 84 | 0.145 857 | 5.56E -19 | 0.558 957 | 0.103 276 | 4.80E -04 | 0.536 88 | 0.076 464 | 0.028 934 |
| MHCflurry (presentation score) | 0.6733 87 | 0.177 378 | 9.75E -25 | 0.556 889 | 0.097 804 | 7.54E -04 | 0.521 614 | 0.073 229 | 0.200 527 |
| NetMHCStabPa n (%Rank_Stab) | 0.5838 7 | 0.122 517 | 5.40E -07 | 0.518 213 | 0.071 701 | 0.280 288 | 0.563 74 | 0.093 54 | 1.61E -04 |
| PRIME (%Rank) | 0.678614 | 0.162947 | 2.20E-26 | 0.558192 | 0.083278 | 4.00E-04 | 0.600326 | 0.09653 | 1.55E-09 |
| DeepHLApan (immunogenic score) | 0.5525 53 | 0.086 783 | 0.001 855 | 0.522 287 | 0.076 852 | 0.186 621 | 0.510 505 | 0.074 257 | 0.533 784 |
| NetMHCphosPa n (Rnk_EL) | 0.6258 28 | 0.142 283 | 9.12E -14 | 0.520 097 | 0.075 307 | 0.233 684 | 0.561 617 | 0.096 367 | 2.62E -04 |
| PIP-EL (inflammatory response) | 0.4609 26 | 0.065 636 | 0.020 728 | 0.474 419 | 0.066 363 | 0.129 793 | 0.473 708 | 0.066 579 | 0.119 485 |
| PROVEAN (functional impact by the mutation | 0.4319 95 | 0.059 677 | 5.64E -05 | - | - | - | - | - | - |
| B-cell linear epitope (BcellPred) | **0.4514 1** | **0.062 508** | **0.004 006** | 0.468 083 | 0.069 809 | 0.058 817 | 0.493 824 | 0.075 081 | 0.714 663 |
| Peptide stability | **0.4555 94** | **0.065 555** | **0.008 785** | 0.486 047 | 0.070 685 | 0.494 792 | 0.479 056 | 0.069 242 | 0.272 637 |
| B-cell conformational epitope (Discotope) | **0.4459 59** | **0.061 71** | **0.001 374** | 0.481 587 | 0.072 856 | 0.276 434 | 0.514 706 | 0.071 238 | 0.383 63 |

Referring to [Table 3], all factors except for TAP efficiency among the factors involved in the biological process for the mutant epitope and epitope properties are statistically significant factors in determining whether an epitope is immunogenic. For DIFFERENCE, among the factors involved in the biological process and the epitope properties, dissimilarity to self-proteome, hydrophobicity, MHCflurry (processing score), MHCflurry (presentation score), and PRIME (%Rank) are statistically significant factors in determining whether an epitope is immunogenic. In addition, for RATIO, among the factors involved in the biological processes and the epitope properties, dissimilarity to self-proteome, NetMHCpan (%Rank_EL), NetMHCpan (%Rank_BA), MHCflurry (processing score), NetMHCStabPan (%Rank_Stab), PRIME (%Rank), and NetMHCphosPan (Rnk_EL) are statistically significant factors in determining whether an epitope is immunogenic.

A total of 30 statistically significant factors (immunogenicity-related biological processes and epitope properties) for the epitopes of the training data set/evaluation data set were converted into a two-dimensional matrix, and machine learning analysis (analysis using an artificial intelligence learning model) was performed. In this Example, several machine learning algorithm analyses were performed using the Weka program, and finally the final model was generated by integrating and selecting several machine learning algorithms. In this Example, the final model was selected based on the average probability of the model results by nine machine learning algorithms: LogitBoost, BayesNet, CSForest, AdaBoost, Logistic, PART, NaiveBayes, RandomForest, and SMO, and in order to compare and evaluate the degree of performance improvement of the present disclosure, published immunogenicity-related programs were performed on the same data and the performances were compared.

[Table 4] below shows the results of the epitope immunogenicity prediction performance evaluation for the training data sets, and [Table 5] shows the results of the epitope immunogenicity prediction performance evaluation for the evaluation data sets.

[Table 4]

| Algorithm | Threshold | Training DB (P:315, N:4067) | | | | | |
|---|---|---|---|---|---|---|---|
| | | F-Measure | Precision | Recall | Accuracy | ROC Area (=AUC) | PRC Area (=prAUC) |
| NetMHCpan (%Rank_EL) | < 0.5 | **0.193** | **0.116** | **0.565** | **0.660** | **0.652** | **0.168** |
| MhcFlurry-2.0 (presentation_score) | > 0.5 | **0.179** | 0.103 | 0.689 | 0.546 | 0.673 | 0.177 |
| PRIME (%Rank) | < 0.5 | **0.210** | 0.131 | 0.524 | 0.716 | 0.679 | 0.163 |
| DeepHLApan (Immunogenic_score ) | > 0.5 | **0.145** | 0.083 | 0.590 | 0.499 | 0.553 | 0.087 |
| IEDB tool | > 0.0 | **0.137** | 0.078 | 0.578 | 0.478 | 0.532 | 0.080 |
| DeepImmuno | > 0.5 | 0.158 | 0.087 | 0.934 | 0.187 | 0.548 | 0.091 |
| TESLA consortium standards | - | 0.194 | 0.164 | 0.238 | 0.858 | 0.572 | 0.228 |
| The present disclosure (NeoPro-Onco) | > 0.5 | 0.302 | 0.259 | 0.362 | 0.880 | 0.724 | 0.234 |

[Table 5]

| Algorithm | Threshold | Independent test DB (P:79, N:3125) | | | | | |
|---|---|---|---|---|---|---|---|
| | | F-Measure | Precision | Recall | Accuracy | ROC Area (=AUC) | PRC Area (=prA UC) |
| NetMHCpan (%Rank_EL) | < 0.5 | 0.285 | 0.176 | 0.747 | 0.908 | 0.942 | 0.317 |
| MhcFlurry-2.0 (presentation_score) | > 0.5 | 0.289 | 0.175 | 0.835 | 0.899 | 0.949 | 0.325 |

(continued)

| Algorithm | Threshold | Independent test DB (P:79, N:3125) | | | | | |
|---|---|---|---|---|---|---|---|
| | | F-Measure | Precision | Recall | Accuracy | ROC Area (=AUC) | PRC Area (=prA UC) |
| PRIME (%Rank) | < 0.5 | 0.323 | 0.208 | 0.722 | 0.925 | 0.919 | 0.328 |
| DeepHLApan (Immunogenic_score) | > 0.5 | 0.059 | 0.031 | 0.468 | 0.629 | 0.538 | 0.032 |
| IEDB tool | > 0.0 | 0.061 | 0.032 | 0.633 | 0.519 | 0.598 | 0.032 |
| DeepImmuno | > 0.5 | 0.110 | 0.058 | 0.892 | 0.139 | 0.608 | 0.099 |
| TESLA consortium standards | - | 0.382 | 0.385 | 0.380 | 0.970 | 0.682 | 0.390 |
| The present disclosure (NeoPro-Onco) | > 0.5 | 0.415 | 0.358 | 0.494 | 0.966 | 0.955 | 0.429 |

In the case of the present disclosure, it is the result of performing 10-fold cross-validation, and in the case of existing published programs and algorithms, it is the result of evaluating the entire data. Referring to [Table 4], it can be confirmed that the method according to the present disclosure shows better prediction accuracy in AUC, prAUC, F-measure, etc. than the existing published program and algorithm methods. Referring to [Table 5], it can be confirmed that the method according to the present disclosure shows better prediction accuracy in AUC, prAUC, F-measure, etc. than the existing published program and algorithm methods even for the evaluation data set that was not used for learning.

[Table 6] shows the results of comparing the performance of a model including all factors and a model excluding DIFFERENCE and RATIO.

[Table 6]

| Algorithm | Threshold | Training DB (P:315, N:4067) | | Independent test DB (P:79, N:3125) | |
|---|---|---|---|---|---|
| | | ROC Area (=AUC) | PRC Area (=prAUC) | ROC Area (=AUC) | PRC Area (=prAUC) |
| Model considering all factors | > 0.5 | 0.724 | 0.234 | 0.955 | 0.429 |
| Model excluding 12 differential indices | > 0.5 | 0.730 | 0.226 | 0.954 | 0.408 |

Since the number of samples for immunogenic/non-immunogenic epitopes is not the same, the comparison was made based on prAUC, which is a more appropriate performance comparison method. Referring to [Table 6], the model including DIFFERENCE and RATIO showed higher performance than the model excluding DIFFERENCE and RATIO, confirming that the Differential index (DIFFERENCE and RATIO) contributes to predicting immunogenic epitopes.

While the inventive concept has been particularly shown and described with reference to exemplary embodiments thereof, it will be understood by those of ordinary skill in the art that various changes in form and details may be made therein without departing from the spirit and scope of the inventive concept as defined by the following claims. It is therefore desired that the embodiments be considered in all respects as illustrative and not restrictive, reference being made to the appended claims rather than the foregoing description to indicate the scope of the disclosure.

**Claims**

1. A method for predicting the immunogenicity of a mutant epitope, comprising:

calculating the degree to which each of factors involved in a biological process for a mutant epitope and properties of the epitope affects the immunogenicity of the mutant epitope;
calculating the degree to which each of factors involved in a biological process for a wild-type epitope corresponding to the mutant epitope and properties of the epitope affects the immunogenicity of the wild-type epitope;

calculate a differential index based on a value calculated for the mutant epitope and a value calculated for the wild-type epitope; and

inputting the value calculated for the mutant epitope and the calculated differential index into a pre-trained artificial intelligence model to predict the immunogenicity of the mutant epitope.

2. The method of claim 1, further comprising performing at least one of standardization and normalization on the value calculated for the mutant epitope and the calculated differential index before inputting the calculated value into the trained artificial intelligence model.

3. The method of claim 1, wherein the differential index includes at least one of RATIO between the value calculated for the mutant epitope and the value calculated for the wild-type epitope and DIFFERENCE between the value calculated for the mutant epitope and the value calculated for the wild-type epitope.

4. The method of claim 1, wherein the differential index is a differential index for at least one of phosphorylation, hydrophobicity, similarity to known epitopes, dissimilarity to self-proteome, epitope stability, antigen processing, MHC I binding affinity, pMHC stability, immunogenicity, inflammatory response, B-cell linear epitope, and B-cell conformational epitope.

5. The method of claim 1, wherein the calculating of the degree to which each of the factors involved in the biological processes for the mutant epitope and the wild-type epitope and the properties of the epitope affects the immunogenicity comprises calculating the degree using an algorithm, a program, or a tool.

6. The method of claim 1, wherein the biological process comprises an antigen processing stage, an antigen presentation stage, an immunogenicity stage, and a tumor microenvironment, and

wherein the factors involved in the tumor microenvironment are at least one of inflammatory response, B-cell linear epitope, and B-cell conformational epitope.

7. A computer-readable recording medium recording a computer program for executing the method of any one of claims 1 to 6.

8. A device for predicting the immunogenicity of a mutant epitope, comprising:

an input/output device receiving a mutant epitope or outputting a prediction result of the immunogenicity of the mutant epitope;

a storage device storing an artificial intelligence model trained to predict the immunogenicity of a mutant epitope using a differential index calculated based on factors involved in biological processes for the mutant epitope and a wild-type epitope and properties of the epitope, and a value calculated based on the factors involved in the biological process for the mutant epitope and the properties of the epitope; and

a computing device calculating the degree to which each of the factors involved in the biological process for the mutant epitope and the properties of the epitope affects the immunogenicity of the mutant epitope, calculating the degree to which each of the factors involved in the biological process for the wild-type epitope corresponding to the mutant epitope and the properties of the epitope affects the immunogenicity of the wild-type epitope, calculating the differential index based on a value calculated for the mutant epitope and a value calculated for the wild-type epitope, and inputting the value calculated for the mutant epitope and the calculated differential index into a pre-trained artificial intelligence model to predict the immunogenicity of the mutant epitope.

# FIG. 1

# FIG. 2

Epitope properties → Antigen processing → Antigen presentation → Immunogenicity → TME

6. Peptide stability

5. Dissimilarity to self-proteome

4. Similarity to known epitopes

3. Hydrophobicity (Physicochemical)

2. Phosphorylation (PTM)

1. Functional impact by mutations

7-2. Antigen processing (TAP transport efficiency)

7-1. Antigen processing (Proteasomal cleavage)

9. pMHC stability

8. MHC class I Binding affinity

10. Immunogenicity (TCR recognition)

13. B-cell conformational epitope

12. B-cell linear epitope

11. Inflammatory response (Pro- / Anti -)

FIG. 3

Predicted value of immunogenicity for epitope

# FIG. 4

Calculating degree to which each of factors involved in biological process for mutant epitope and properties of epitope affects immunogenicity of mutant epitope — S410

Calculating degree to which each of factors involved in biological process for wild-type epitope corresponding to mutant epitope and properties of epitope affects immunogenicity of wild-type epitope — S420

Calculating differential index based on value calculated for mutant epitope and value calculated for wild-type epitope — S430

Inputting value calculated for mutant epitope and calculated differential index into pre-trained artificial intelligence model to predict immunogenicity of mutant epitope — S440

# FIG. 5

## INTERNATIONAL SEARCH REPORT

| International application No. |
|---|
| **PCT/KR2023/017786** |

**A. CLASSIFICATION OF SUBJECT MATTER**

**G16B 5/00**(2019.01)i; **G16B 20/20**(2019.01)i; **G16B 40/00**(2019.01)i

According to International Patent Classification (IPC) or to both national classification and IPC

**B. FIELDS SEARCHED**

Minimum documentation searched (classification system followed by classification symbols)

G16B 5/00(2019.01); G16B 20/00(2019.01); G16B 20/20(2019.01); G16B 30/00(2019.01); G16B 40/00(2019.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 신생항원(neoantigen), 에피토프(epitope), 면역원성(immunogenicity), 돌연변이 (mutation), 비율(ratio), 차이(difference), 인덱스(index), 원시(wild), 인공지능 모델(artificial intelligence model)

**C. DOCUMENTS CONSIDERED TO BE RELEVANT**

| Category* | Citation of document, with indication, where appropriate, of the relevant passages | Relevant to claim No. |
|---|---|---|
| A | SCHMIDT, J. et al. Prediction of neo-epitope immunogenicity reveals TCR recognition determinants and provides insight into immunoediting. Cell Reports Medicine. 16 February 2021, vol. 2, thesis no. 100194, pp. 1-14, Star Methods (e1-e7). <br><br> See entire document. | 1-8 |
| A | SMITH, C. C. et al. Machine-learning prediction of tumor antigen immunogenicity in the selection of therapeutic epitopes. Cancer Immunology Research. 2019, vol. 7, no. 10, pp. 1591-1604. <br> See entire document. | 1-8 |
| A | KR 10-2399419 B1 (BIONTECH SE et al.) 19 May 2022 (2022-05-19) <br> See entire document. | 1-8 |
| A | GARTNER, J. J. et al. A machine learning model for ranking candidate HLA class I neoantigens based on known neoepitopes from multiple human tumor types. Nature Cancer. May 2021, vol. 2, pp. 563-574. <br> See entire document. | 1-8 |

☑ Further documents are listed in the continuation of Box C.     ☑ See patent family annex.

| | | |
|---|---|---|
| \* | Special categories of cited documents: | "T" later document published after the international filing date or priority date and not in conflict with the application but cited to understand the principle or theory underlying the invention |
| "A" | document defining the general state of the art which is not considered to be of particular relevance | |
| "D" | document cited by the applicant in the international application | "X" document of particular relevance; the claimed invention cannot be considered novel or cannot be considered to involve an inventive step when the document is taken alone |
| "E" | earlier application or patent but published on or after the international filing date | |
| "L" | document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" | document referring to an oral disclosure, use, exhibition or other means | |
| "P" | document published prior to the international filing date but later than the priority date claimed | "&" document member of the same patent family |

| Date of the actual completion of the international search | Date of mailing of the international search report |
|---|---|
| **08 February 2024** | **08 February 2024** |

| Name and mailing address of the ISA/KR | Authorized officer |
|---|---|
| **Korean Intellectual Property Office** <br> **Government Complex-Daejeon Building 4, 189 Cheongsa-** <br> **ro, Seo-gu, Daejeon 35208** | |
| Facsimile No. **+82-42-481-8578** | Telephone No. |

Form PCT/ISA/210 (second sheet) (July 2022)

**INTERNATIONAL SEARCH REPORT**

International application No.

**PCT/KR2023/017786**

### C.    DOCUMENTS CONSIDERED TO BE RELEVANT

| Category* | Citation of document, with indication, where appropriate, of the relevant passages | Relevant to claim No. |
|---|---|---|
| A | KR 10-2020-0109269 A (SYNTEKABIO CO., LTD.) 22 September 2020 (2020-09-22)<br>See entire document. | 1-8 |

Form PCT/ISA/210 (second sheet) (July 2022)

## INTERNATIONAL SEARCH REPORT
### Information on patent family members

International application No.

**PCT/KR2023/017786**

| Patent document cited in search report | | | Publication date (day/month/year) | Patent family member(s) | | | Publication date (day/month/year) |
|---|---|---|---|---|---|---|---|
| KR | 10-2399419 | B1 | 19 May 2022 | AU | 2014-264943 | A1 | 26 November 2015 |
| | | | | AU | 2014-264943 | B2 | 12 September 2019 |
| | | | | AU | 2019-275637 | A1 | 02 January 2020 |
| | | | | AU | 2022-200863 | A1 | 03 March 2022 |
| | | | | CA | 2911945 | A1 | 13 November 2014 |
| | | | | CA | 2911945 | C | 17 October 2023 |
| | | | | CN | 105451759 | A | 30 March 2016 |
| | | | | CN | 105451759 | B | 05 February 2021 |
| | | | | CN | 113219179 | A | 06 August 2021 |
| | | | | EP | 2994159 | A1 | 16 March 2016 |
| | | | | EP | 2994159 | B1 | 10 March 2021 |
| | | | | EP | 3895727 | A1 | 20 October 2021 |
| | | | | JP | 2016-521128 | A | 21 July 2016 |
| | | | | JP | 2020-103297 | A | 09 July 2020 |
| | | | | JP | 2021-121801 | A | 26 August 2021 |
| | | | | JP | 6710634 | B2 | 17 June 2020 |
| | | | | JP | 6882550 | B2 | 02 June 2021 |
| | | | | JP | 7405792 | B2 | 26 December 2023 |
| | | | | KR | 10-2016-0030101 | A | 16 March 2016 |
| | | | | US | 11222711 | B2 | 11 January 2022 |
| | | | | US | 2016-0125129 | A1 | 05 May 2016 |
| | | | | US | 2022-0093209 | A1 | 24 March 2022 |
| | | | | WO | 2014-180490 | A1 | 13 November 2014 |
| | | | | WO | 2014-180569 | A1 | 13 November 2014 |
| | | | | ZA | 201508048 | B | 31 May 2017 |
| KR | 10-2020-0109269 | A | 22 September 2020 | KR | 10-2406699 | B1 | 08 June 2022 |
| | | | | US | 2022-0130489 | A1 | 28 April 2022 |
| | | | | WO | 2020-185010 | A1 | 17 September 2020 |

Form PCT/ISA/210 (patent family annex) (July 2022)